# DEMANDE DE BREVET EUROPEEN

(11) **EP 1 759 725 A1**
(43) Date de publication de la demande: **07.03.2007**
(21) Numéro de dépôt: 05447197.4
(22) Date de dépôt: 31.08.2005
(51) Int. Cl.: A61M 1/00, A61M 31/00, A61B 5/00

(54) **Sonde electro-biochimique d'analyse intramusculaire ou intracerebrale**

(71) Demandeur: Université Libre de Bruxelles, 1050 Brussels (BE)
(72) Inventeur: Manto, Mario, 1440 Braine-Le-Chateau (BE)
(74) Mandataire: Van Malderen, Joëlle

(57) **Abrégé**

La présente invention se rapporte à une sonde de microdialyse intramusculaire ou intracérébrale avec un guide (1) comprenant :
- au moins une chambre (2) avec des pores (5), ladite chambre (2) étant connectée à un élément d'infusion (3) d'un liquide vers ladite chambre (2) et un élément d'aspiration (4) dudit liquide de ladite chambre (2) et,
- des électrodes d'enregistrement (7,8) et de stimulation (9) capables de délivrer des différences de potentiel continu.

## Description

### Objet de l'invention

La présente invention est relative à une sonde électro-biochimique d'analyse, de préférence intramusculaire ou intracérébrale et à tout dispositif ou appareillage comprenant ladite sonde.

### Arrière-plan technologique et état de la technique à la base de l'invention

Le tissu musculaire est un tissu électriquement excitable. Les fibres musculaires produisent des courants électriques lorsqu'elles se contractent. En clinique humaine, ces courants sont analysés par la technique de l'électromyographie à l'aiguille ou de surface. Le muscle est aussi le siège de nombreux échanges métaboliques. Par exemple, lors de contractions musculaires prolongées (comme des efforts soutenus), il y a production d'acide lactique.

En pathologie humaine, de nombreuses maladies sont caractérisées par une production anormale de métabolites dans le muscle. Ainsi, dans les maladies mitochondriales, la production d'acide lactique est anormale. La technique la plus utilisée pour analyser les échanges métaboliques in vivo au niveau du tissu interstitiel est la microdialyse.

Cette technique ne nécessite de collecter que quelques microlitres du tissu intercellulaire (de l'ordre de 10 à 20 microlitres). Le liquide est collecté grâce à une membrane semi-perméable insérée dans le muscle via une cannule-guide. L'élément tubulaire du dispositif est connecté à une pompe de micro-perfusion et les métabolites diffusent du milieu intercellulaire à travers une sonde. Les molécules étudiées ont en général un poids moléculaire de moins de 80 kDaltons. Initialement, la technique de microdialyse a été mise au point pour mesurer les concentrations de neurotransmetteurs dans le cerveau (Ungerstedt U. Microdialysis - principles and applications for studies in animals and men. J. Intern. Med. 1991 ; vol. 230, p. 365-373). Dans le dispositif de l'état de la technique, la membrane semi-perméable est une membrane en cuprophane.

Les exemples d'applications actuelles de ces sondes de microdialyse sont le suivi de la présence et/ou de l'action de métabolites intracrâniens chez des patients dans le coma (par exemple, l'augmentation des taux de glycérol témoigne de lésions des membranes neuronales) et l'évaluation des effets de nouveaux agents pharmacologiques chez les rats, lors d'études pré- cliniques (Muller M. Microdialysis. BMJ 2002; vol.324, p. 588-591).

### Buts de l'invention

La présente invention vise la mise au point d'une nouvelle sonde permettant de caractériser de manière originale, non seulement le comportement électrique d'un muscle ou du cerveau d'un patient, mais également simultanément les échanges métaboliques au sein du muscle du cerveau.

La présente invention vise en particulier à fournir une telle sonde ou tout dispositif la comprenant, pour améliorer l'étude, le diagnostic, le suivi et la thérapie de différentes pathologies dont peuvent souffrir différents animaux, en particulier les mammifères, et plus particulièrement l'homme.

### Eléments caractéristiques de l'invention

La présente invention est relative à une sonde de microdialyse de préférence intramusculaire comprenant un guide pourvu d'au moins une chambre, dite « terminale », la chambre étant connectée à un élément d'infusion d'un liquide vers la chambre et connectée à un élément d'aspiration dudit liquide de la chambre. La sonde comporte également des électrodes d'enregistrement et de stimulation capables d'enregistrer des potentiels d'action et/ou de délivrer des différences de potentiels continus, de préférence au niveau du muscle dans lequel elle est insérée.

Selon une forme d'exécution préférée de l'invention, la sonde comprend plusieurs chambres, chaque chambre étant connectée de préférence de manière séparée à un élément d'infusion d'un liquide vers ladite chambre et à un élément de circulation ou d'aspiration dudit liquide vers ladite chambre. Ces multiples chambres sont de préférence formées par des prismes ou des cylindres présentant une section d'un diamètre décroissant, insérés les uns dans les autres et éventuellement coulissables les uns par rapport aux autres. Par conséquent, les parois de ces chambres sont formées par les parois de ces différents prismes ou cylindres.

Cette forme d'exécution permet de déplacer les différentes chambres des sondes jusqu'à une zone d'intérêt. En effet, dans les applications intramusculaires, on peut générer de manière plus efficace des différences de potentiels continus et d'enregistrement de potentiels d'action, au niveau de ces muscles, ainsi que réaliser de manière efficace les opérations de dialyse ou d'administration d'agents thérapeutiques.

De même, il est également possible d'utiliser de telles sondes, de manière à déplacer une ou plusieurs chambres vers une zone d'intérêt du cerveau. Dans le cas d'une application cérébrale, ceci est particulièrement important car le cortex cérébral est caractérisé par des sillons qui varient anatomiquement et ces variations anatomiques sont très peu prévisibles et varient en fonction du sujet traité (animal non humain ou humain). L'adaptation d'une sonde en fonction de ces différentes caractéristiques génère une perte de temps considérable. Par conséquent, la sonde de l'invention permet de s'adapter à ces configurations individuelles. Le déplacement des différentes chambres (par coulissement des prismes ou cylindres formant ces chambres) peut se faire par des moyens connus de l'homme de l'art et qui peuvent être également incorporés à la sonde de l'invention.

De préférence, dans la sonde de l'invention, soit chaque chambre comporte des pores de taille différente, dont la taille est différente d'une chambre à l'autre, ou soit chaque chambre peut comporter différents pores de taille différente. Ces deux configurations permettent l'administration de différents composés, soit faite depuis des chambres différentes ou soit depuis la même chambre, mais libérés en fonction de la taille des pores.

Néanmoins, si l'on cherche à collecter simultanément des molécules de tailles différentes, on choisira d'utiliser une chambre présentant des pores de taille différente par rapport aux pores existant dans une autre chambre, cette autre chambre étant destinée à recueillir d'autres molécules avec une autre taille.

Selon une autre forme d'exécution préférée de l'invention, la sonde de l'invention comporte également une ou plusieurs fibres optiques communiquant avec la ou les chambre(s). De préférence, la sonde de l'invention comporte un senseur de température ou une fibre optique par chambre, chaque fibre étant munie d'un éclairage à intensité variable. En outre, la fibre optique pourra comporter de préférence à son extrémité un senseur de force. Un senseur de force peut par exemple être disposé à l'extrémité de la fibre optique. Ce senseur de force peut comporter soit des inclusions d'autres éléments de fibres optiques, soit une ou plusieurs bulles de gaz susceptibles de subir des déformations, ces déformations étant détectées par le senseur de force et transmises à travers la fibre optique.

La présente invention est également relative à un dispositif comprenant une ou plusieurs sondes de l'invention connectées entre elles et comportant un élément créateur d'un champ magnétique. Ce champ magnétique est formé à côté d'au moins une des chambres. De préférence, ce champ magnétique est obtenu par un courant électrique induit dans des spires d'un fil électrique, lesdites spires étant présentes dans au moins une des chambres.

De manière avantageuse, le dispositif est constitué d'au moins deux sondes et d'un élément d'injection (piston) d'un liquide depuis une des sondes. Ce dispositif permet l'introduction d'un liquide depuis une sonde vers une autre sonde proche de la première. L'injection à partir d'une des sondes du liquide vers un milieu interstitiel (intramusculaire), permet de recueillir, au niveau de la seconde sonde, une portion du liquide mis en contact avec le liquide interstitiel (intramusculaire) et permet de recueillir les molécules présentes dans ce dit liquide interstitiel.

Un autre aspect de l'invention concerne un dispositif comprenant au moins une des sondes de l'invention, un élément d'amplification et de filtrage des signaux électriques provenant des électrodes d'enregistrement de la sonde, un élément d'analyse de données biochimiques obtenues sur le liquide d'aspiration provenant de la chambre de la sonde et un écran permettant un affichage simultané des signaux électriques et des données biochimiques obtenues de la sonde.

Un autre aspect de l'invention concerne un appareillage comprenant ledit dispositif décrit ci-dessus, combiné à un dispositif d'analyse d'un électroencéphalogramme (EEG) ou d'un électromyogramme (EMG).

La présente invention est également relative à un dispositif comprenant un alignement successif ou un réseau (array) de sondes comprenant de multiples chambres, de multiples éléments d'infusion et d'aspiration d'un ou de plusieurs liquides identique(s) ou différent(s) selon les chambres et de multiples électrodes d'enregistrement et de stimulation (accolées ou présentes dans les chambres).

### Description détaillée de l'invention

Lors d'une consultation de neurologie, il est important que l'examinateur dispose en temps réel et de manière instantanée des données électriques et métaboliques d'un muscle ou au niveau cérébral, par l'utilisation d'une sonde d'analyse électro-biochimique intramusculaire ou cérébrale.

Cette analyse immédiate permet de caractériser les indications cliniques générales suivantes, chez un animal ou un patient humain :
- évaluation des effets de la stimulation électrique sur la production de métabolites in loco ;
- diagnostiquer les maladies musculaires métaboliques du patient par simulation de contractions prolongées des fibres musculaires ;
- étude de la jonction neuromusculaire d'une fibre musculaire isolée ;
- étude simultanée de plusieurs jonctions neuromusculaires analysées simultanément tant sur le plan électrique que biochimique.

Cependant, à l'heure actuelle, il n'existe pas de sonde d'analyse électro-biochimique capable de fournir de manière instantanée les informations sur un signal électrique (électromyographie ou électroencéphalographique) et un signal biochimique (analyse des métabolites) provenant d'une fibre musculaire ou du cerveau.

Une telle sonde électro-biochimique avec de multiples chambres est capable d'analyser les comportements électriques « étagés » de fibres musculaires superposées ou d'une sonde à multiples compartiments, permettant l'administration simultanée au muscle ou au cerveau de différents composés actifs, chacun des composés étant administré depuis une chambre différente à proximité d'une ou plusieurs fibres musculaires ou du cerveau.

Selon une forme d'exécution préférée de l'invention, la sonde comprend de multiples chambres, insérées les unes dans les autres. Des chambres insérées sont par exemple constituées de prismes ou de cylindres insérés avec des sections de diamètre décroissant. Les prismes ou cylindres sont insérés (imbriqués) les uns dans les autres, afin de permettre le coulissement d'une chambre par rapport à une autre.

Selon une forme d'exécution préférée de l'invention, les électrodes d'enregistrement et de stimulation sont collées ou insérées dans les parois externes desdites chambres.

L'invention concerne aussi un appareillage ou un dispositif comportant une telle sonde et qui permet d'améliorer le diagnostic de maladies neuronales (neuromusculaires ou cérébrales) par une analyse électro-biochimique très sensible afin d'évaluer, avantageusement et de manière directe et simultanée, l'activité électrique et biochimique des fibres musculaires ou du cerveau. Cette sonde et ce dispositif permettent d'évaluer les effets de stimulation électrique (intramusculaire, ou intracérébrale) sur la production de métabolites (au sein d'un muscle ou du cerveau) et ceci de manière « étagée » ou non, et ensuite de comparer de manière simultanée l'activité de plusieurs fibres musculaires ou du cerveau avec les activités électromyographiques de surface ou électroencéphalographiques du cerveau, lors de l'administration de composés actifs (en particulier des médicaments administrés dans le muscle ou dans le cerveau). De plus, un tel dispositif ou une telle sonde, permettent de réaliser des analyses électro-biochimiques compartimentées, in vivo, chez l'animal, en particulier chez le mammifère, plus particulièrement chez un patient humain, à l'état éveillé ou non.

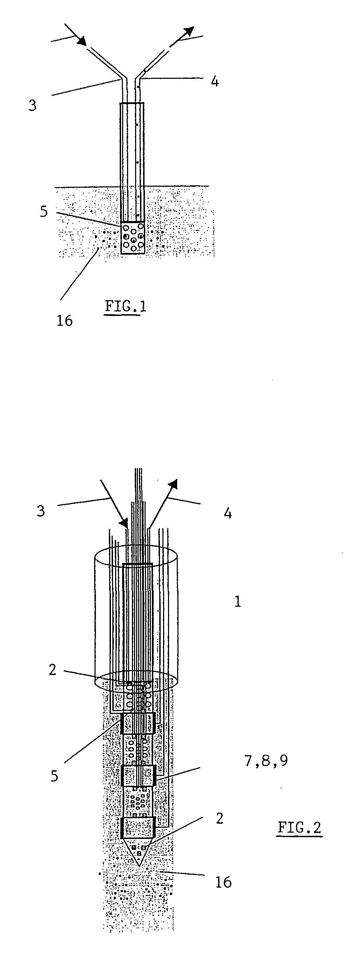

Les sondes de dialyses classiques actuellement utilisées, telles que représentées à la figure 1, comportent les éléments suivants :
- un élément d'infusion (3) d'un liquide de composition connue;
- un élément d'aspiration (4) dudit liquide, permettant l'analyse dudit liquide en sortie;
- des pores (5) en contact avec le liquide interstitiel (16).

Contrairement aux sondes de dialyse classiques, la présente invention concerne une sonde électro-biochimique d'analyse (intramusculaire ou intracérébrale) (voir figure 2) comportant les éléments suivants :
- un guide (1) comportant une pointe terminale et une chambre « terminale » (2) comprenant des pores (5),
- des membranes semi-perméables,
- une pompe capable d'infuser un liquide à un débit de 0.5 à 2 *µ*l/min,
- un élément d'infusion (3) dudit liquide (via la pompe) vers la chambre (2) et un élément d'aspiration (4) d'un liquide depuis ladite chambre (2),
- des électrodes de stimulation (9) et d'enregistrement (7,8),
- différents stimulateurs électriques,
- des préamplificateurs et,
- des filtres "low-pass" et "high-pass" pour l'analyse électrique des électrodes.

De préférence, contrairement aux sondes de dialyse de l'état de la technique, la sonde de l'invention présente un modèle dit « compartimenté » à plusieurs (trois) chambres étagées (2,2',2") comportant pour chaque chambre (2,2',2") des pores (5) (de préférence de taille différente) qui permettent de sélectionner les molécules à analyser.

Les chambres de la sonde de l'invention peuvent avoir une forme cylindrique ou prismatique à section hexagonale ou octogonale. La forme cylindrique présente l'avantage d'être moins traumatisante lors de l'insertion de la sonde, mais la forme prismatique présente l'avantage d'être plus simple à réaliser. Comme les pores sont de taille différente ; on peut tester l'effet d'une même drogue à trois concentrations différentes présentes dans les trois chambres de la sonde de l'invention.

En outre, si on administre ou si on analyse des molécules de taille différente, on pourra également utiliser une sonde avec des chambres présentant des pores de taille différente destinées à permettre la circulation et l'aspiration de différentes molécules.

Dans ce dispositif, chaque chambre (2,2',2") est de préférence connectée à des d'éléments d'infusion (3) et de circulation ou d'aspiration (4) différents, de manière à permettre l'injection de molécules différentes ou l'analyse de molécules différentes en fonction des chambres.

Par conséquent, le liquide infusé par l'élément (3) au niveau d'une chambre (2) peut comporter des agents thérapeutiques particuliers libérés seulement au niveau des pores (5) d'une des chambres, ou ce liquide peut être un liquide provenant du milieu interstitiel (intramusculaire ou intracérébral) soumis ensuite à une analyse des molécules susceptibles d'être présentes dans ledit milieu interstitiel.

Ces molécules entraînées par l'élément (4) d'aspiration dudit liquide sont traitées par un dispositif pour permettre leur identification et leur quantification (de préférence en continu) par des moyens d'analyse biochimiques connus de l'homme de l'art.

L'infusion et l'aspiration sont enclenchées par une pompe capable d'infuser un liquide à un débit de 0,5 à 2 *µ*l/min au niveau d'une chambre.

Cette sonde, telle que représentée à la figure 2 comporte également des électrodes d'enregistrement (7,8) et des électrodes de stimulation (9) disposées entre chaque chambre, accolées à chaque chambre ou présentes dans chaque chambre. Ces électrodes de stimulation (9) sont capables d'enregistrer des potentiels d'action et/ou de délivrer des différences de potentiel continu qui vont créer des variations au seuil des décharges électriques des fibres musculaires. Les électrodes d'enregistrement peuvent être par exemple incorporées dans une première chambre, de manière à détecter les potentiels de dénervation (dans une zone de muscle la plus superficielle). En effet, cette zone constitue une interface avec le tissu conjonctif (qui entoure le muscle). Dès lors, certaines maladies de cette zone sont plus facilement détectées. On peut toutefois associer à chaque chambre des électrodes d'enregistrement et de stimulation.

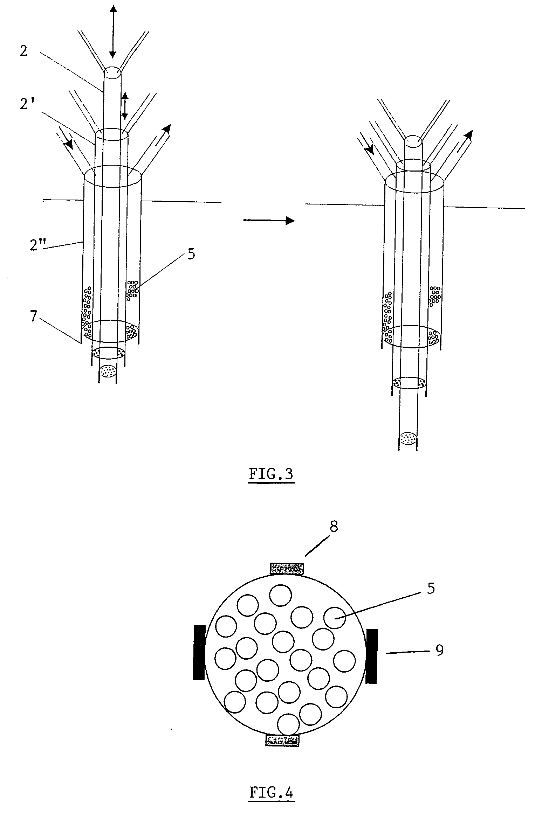

Une variante d'exécution de la sonde de l'invention ayant la forme d'une « longue-vue » est représentée à la fig. 3. Une forme dite « longue-vue » est constituée de plusieurs chambres (2, 2', 2") susceptibles de coulisser les unes par rapport aux autres. Comme représenté à la fig. 3, la sonde électro-biochimique est composée de trois cylindres insérés les uns dans les autres avec un diamètre décroissant. Il est également possible d'envisager une telle sonde avec des prismes de section octogonale ou hexagonale décroissante. Les parois des cylindres ou prismes permettent la formation de trois chambres, chaque chambre comportant un élément d'infusion et un élément d'aspiration différent.

De manière avantageuse, à l'extrémité de chaque cylindre se trouvent au moins deux électrodes qui servent soit à l'enregistrement de potentiels électriques, soit à la stimulation de fibres musculaires ou des deux en alternance. Les électrodes sont, de préférence, collées contre la paroi du cylindre ou du prisme ou insérées dans la paroi du cylindre ou du prisme. Tel que représenté à la fig. 3, chacune des chambres peut comporter des pores de taille différente. Ces pores pouvant être éventuellement regroupées en grappe.

Un examinateur peut analyser des potentiels électriques au niveau des électrodes de chacun des cylindres. Dans ce cas, il peut découvrir une zone électriquement active et infusée du liquide dans les trois chambres et analyser le microdialysat récolté. Si un potentiel électrique est détecté uniquement au niveau d'une des chambres de la sonde, il est possible par une manipulation manuelle visant à faire coulisser un cylindre et à rapprocher la telle sonde d'une zone musculaire à étudier. Cette sonde permet de concentrer des molécules de petite faille qui seraient libérées dans un milieu intercellulaire (entre les fibres musculaires) suite à une stimulation électrique et de pouvoir déterminer quelle est la zone (du muscle) étudiée où ce phénomène est le plus marqué (par exemple, la zone du muscle qui traduit une vulnérabilité musculaire).

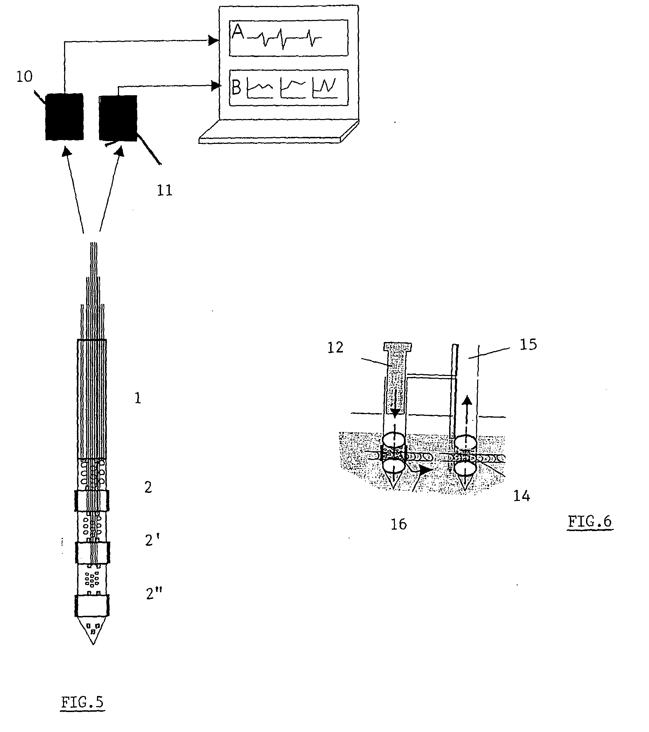

La fig. 4 représente une coupe transversale de l'extrémité d'un des cylindres de la sonde de la fig. 3. Celle-ci représente les pores disposés à l'extrémité du cylindre ainsi que les électrodes de stimulation et d'enregistrement disposées en alternance sur l'extrémité du cylindre. En dissociant les sites d'enregistrement et de stimulation, il est possible d'enregistrer et de stimuler en continu tout en gardant la possibilité d'explorer dans l'espace le contenu du microdialysat aspiré au niveau de chaque chambre.

La sonde de l'invention peut comporter également un senseur de variation de la température et/ou une ou plusieurs fibres optiques communiquant avec la ou les chambres. De préférence, les fibres optiques sont par exemple des fibres optiques de l'ordre de 590 microns tel que commercialisé par la Société WPI. Le changement de pression enregistré dans une chambre, de préférence la chambre disposée à l'extrémité de la sonde induit un changement de taille locale au niveau de la fibre optique qui modifie les caractéristiques du faisceau lumineux dans ladite fibre (principe d'interférométrie lumineuse).

De manière avantageuse, on incorpore une fibre optique différente dans chaque chambre et chaque fibre est munie d'un éclairage à intensité variable. Selon une autre forme d'exécution de l'invention, la fibre optique peut être accolée à l'extérieur d'une chambre en vue de visualiser la morphologie du cerveau ou des fibres musculaires analysées et si possible la dynamique de contraction des fibres musculaires ou l'absence de contraction en cas de fibrose locale, d'élongation musculaire chez un sportif ou d'hématomes. Pour cela, un système de type caméra endoscopique peut être couplé aux fibres optiques, telle que la caméra PSV-2705^{™} (Optovision France, Toulouse). Il est également possible d'utiliser des fibres permettant de détecter des anomalies de teinte ou de densité de couleurs, (tel que le produit IV-C35 du système de vision couleurs de la Société Hoskin).

L'analyse spectrométrique du signal lumineux passant par la fibre optique permet de révéler des anomalies spécifiques (par exemple, en cas de fibrose musculaire vu la texture, la composition et la perte d'élasticité du tissu musculaire analysé). Il est également possible d'incorporer un senseur de force à l'extrémité de la fibre optique. Pour « palper » la surface des fibres musculaires et détecter des anomalies comme des zones de fibrose ou les caractéristiques de fibres présentant une morphologie déchiquetée. De plus, toute déformation de ces fibres optiques ou de la sonde comprenant cette fibre optique est détectée au niveau de la fibre optique et fournit des informations sur la dynamique de contractions de fibres musculaires analysées.

Différents dispositifs d'analyses électro-biochimiques hybrides (« AEBH ») incorporant ce dispositif permettent d'afficher simultanément sur un écran du dispositif, tel que représenté à la fig. 5, les caractéristiques électriques et biochimiques d'un muscle ou des portions d'un même muscle, ou même de muscles différents (s'ils sont superposés) lors d'une consultation en neurologie. Ce dispositif invasif permettant l'analyse instantanée et simultanée de potentiels électriques intramusculaires et de dosages biochimiques de molécules ou substances, dans la zone du muscle ou du cerveau étudié sur le plan électrique. Par conséquent, le dispositif de l'invention permet de délivrer des courants électriques in loco et d'apprécier de préférence par la technique de visualisation intramusculaire la morphologie des fibres musculaires et leur dynamique de contractions.

Dans ce cas, le dispositif comprend un élément (10) d'amplification et de filtrage du signal électrique, un élément (11) permettant une analyse du signal biochimique et un écran permettant un affichage simultané des signaux électriques (A) et des données biochimiques (B).

Parmi les données biochimiques que l'on peut collecter et visualiser au moyen du dispositif de l'invention au niveau musculaire, on peut citer notamment le dosage d'acide lactique, d'acide nitrique, d'acide pyruvique, d'adrénaline/noradrénaline, d'ATP, de glutamate/glutamine, de taux d'ions (chlore, potassium, calcium, sodium), d'acides aminés, de glucose, de glycérol, de methoxy 4-hydroxyphenylglycol (MHPG), d'acétylcholine ou d'interleukines. Au niveau cérébral, on peut doser l'oxide nitrique (marqueur de toxicité neuronale) combiné au suivi de la température intracérébrale. En effet, ce dernier paramètre apparaît comme important pour la protection des neurones cérébraux (Georgiadis D. et al. « Hypothermia in acute stroke », Curr. Treat. Options Neurol. 2005, vol. 7 (2) p. 119 - 127). Parmi les signaux électriques que l'on peut visualiser au moyen du dispositif de l'invention, il est possible d'obtenir les potentiels de dénervation, des potentiels d'unités motrices, des potentiels tardifs, des potentiels collatéraux en cas de ré-innervation, de fasciculations, de décharges dites « bizarres » (complex high-frequency discharges), de rafales myotoniques traduisant une hyperactivité musculaire, etc.

On peut détecter des potentiels électriques de morphologie complexe qui apparaissent dans des maladies dites maladies des motoneurones inférieurs ou dans des maladies musculaires de type myopathie (Ref. Peripheral Neurology par Jay Liveson, F.A. Davis, Philadelphia, 1991). Il est également possible de tester la concentration d'un composé particulier présent dans la chambre et de permettre que la concentration d'un composé, tel que l'oxide nitrique puisse provoquer une réaction colorimétrique s'affichant sur l'écran (en particulier, un écran réalisé en un matériau polymérique flexible, tel qu'un écran OLED). Des cofacteurs, par exemple nécessaires dans la chambre ou dans le dispositif, ainsi que des enzymes peuvent être utilisés pour obtenir une réaction colorimétrique pour doser l'oxide nitrique.

En administrant simultanément trois médicaments différents (par exemple, dans le cas d'une sonde multi-compartimentée), on pourra choisir celui qui améliore au mieux la fonction musculaire ou cérébrale, tant sur le plan électrique que sur le plan biochimique.

Cette administration simultanée de trois médicaments différents se faisant par les (différents) pores de la sonde de l'invention.

En outre, le dispositif de l'invention peut comporter des éléments supplémentaires permettant une analyse directe du liquide interstitiel des muscles ou du cerveau en cas d'urgence (par exemple en cas d'accident de la route avec un patient comprimé par un véhicule, tremblement de terre,...). Cette analyse se fera par une sonde permettant de créer un champ magnétique local.

Dans ce cas, le dispositif comporte deux sondes, la première sonde de l'invention, telle que représentée à la figure 6, comporte un élément pour une injection manuelle (piston (12)) d'un liquide dans le milieu interstitiel (16), des spires (14) présentes dans les chambres de la sonde et une seconde sonde (15) permettant de recueillir du liquide provenant du milieu interstitiel (16) afin de permettre une analyse immédiate des caractéristiques de ce milieu.

Dans la sonde, les spires (14) sont utilisées pour créer un champ magnétique dit « local». Les fibres musculaires pouvant être dépolarisées de manière soutenue, grâce à ce champ magnétique créé par un courant électrique dans les spires (14).

De préférence, le champ magnétique est provoqué par un courant électrique introduit dans un fil métallique flexible d'un diamètre de l'ordre de 0,6 mm formant des spires. 500 spires sont utiles pour créer un champ magnétique dit local, capable de dépolariser entre 3 et 4 fibres musculaires contiguës.

Il est également possible pour l'homme du métier d'adapter les caractéristiques du fil métallique, de manière à augmenter le champ magnétique créé par le courant électrique (réduction ou augmentation du nombre de spires, modification des caractéristiques du fil métallique, modification du courant induit, ...).

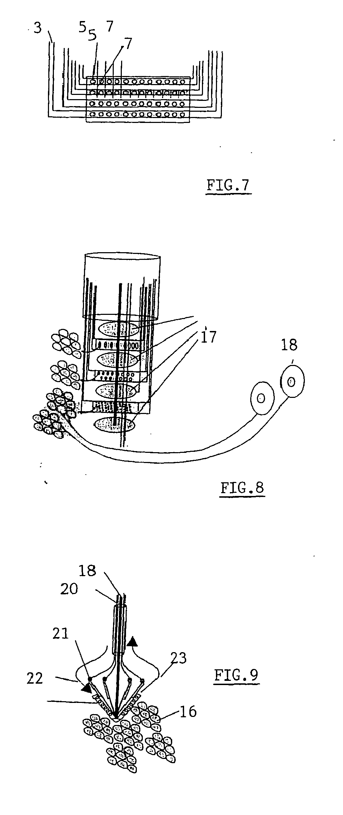

Actuellement, un des problèmes importants est de ne pas pouvoir analyser le comportement électro-biochimique de fibres musculaires qui sont alignées en parallèle. Pour résoudre ce problème, le dispositif de l'invention est prévu selon un réseau (array) de plusieurs sondes, tel que représenté à la figure 7 et qui est placé en surface du muscle qui est exposé (suite à un traumatisme, une brûlure, ou suite à une dissection chez un animal de laboratoire). Le réseau comporte un (ou plusieurs) moyen(s) d'infusion (3), des pores (5) et des électrodes (7) pour un enregistrement et une stimulation.

Cette configuration en alignement ou en réseau a l'avantage de pouvoir analyser le comportement de chacune des fibres musculaires, lorsque des médicaments différents sont infusés dans chacune des voies de circulation du liquide. Il est ensuite possible d'effectuer une analyse instantanée des décharges électriques de chacune des fibres soumises à des drogues différentes.

Selon l'invention, on utilise de préférence des électrodes en titanium flexible dont l'extrémité est d'environ 5 microns (par exemple des fils portant la référence 500449 de la Société WPI), des fils en carbone de diamètre d'environ 30 microns ou des fils en platinium-iridium de diamètre d'environ 0,051 mm (référence PT0203 de WPI). Ces électrodes sont disposées de manière latérale le long des chambres de la sonde, chacune des chambres présente un diamètre compris de préférence entre environ 50 et environ 250 microns (afin de pouvoir étudier plusieurs fibres musculaires). La hauteur d'une chambre est de préférence comprise entre environ 200 à environ 500 microns. Cependant, des chambres de hauteur plus importante, de l'ordre du mm peuvent être aussi conçues, si l'on souhaite étudier les caractéristiques du liquide interstitiel dans des situations particulières (hématomes). Dans ce cas, le liquide interstitiel est plus hétérogène et nécessitera une étude plus complexe par des chambres de plus grande taille. Dans un alignement ou un réseau, les sondes sont espacées entre elles par une distance comprise entre environ 250 microns et environ 2 mm. Cette variation dépend de la pathologie investiguée. Lorsque le dispositif comprend l'utilisation de sondes à multiples compartiments (chambres), le réseau permet par exemple d'étudier des fibres d'un même fascicule ou de fascicules différents (dans certaines pathologies, seuls certains fascicules sont atteints). De plus, une sonde à multiples compartiments (multiples chambres) est particulièrement utile pour étudier de préférence des fibres dans un même alignement. Cette analyse peut se faire de manière directe par une seule introduction de la sonde, contrairement au système des aiguilles standards pour une analyse électromyographique ou électroencéphalographique qui doit être insérée et ressortie à plusieurs reprises. Cette analyse permet donc d'éviter de multiples piqûres qui peuvent se révéler douloureuses pour le patient. Ce dispositif permet également d'étudier les maladies musculaires ou cérébrales qui affectent le muscle ou le cerveau de manière hétérogène.

La figure 8 représente l'étude du comportement de faisceaux (groupes) de fibres musculaires lorsque des différences de potentiels constants sont imposées entre les différentes chambres permettant la collecte du liquide.

Dans la figure 8, on représente les plateaux électriques soumis à des différences de potentiels stables (17) et les motoneurones (18) innervant les fibres musculaires. De préférence, pour cette application, les pores de la chambre dans la sonde de l'invention sont de taille différente.

La figure 9 représente une sonde qui permet d'analyser les échanges métaboliques au niveau de fibres musculaires appartenant à des faisceaux contigus et de connaître simultanément les activités électriques des fibres à distance. La sonde comprend une électrode de référence (18), des électrodes actives (20) et une zone d'enregistrement de l'activité électrique (21). Les directions inflow (22) et outflow (23) sont également représentées sur la figure 9 par la référence (24).

Une telle sonde dite « parapluie » peut s'ouvrir par pression, telle que représentée sur la figure. Lorsque le parapluie est fermé, les éléments constitués par la sonde d'enregistrement sont à une certaine distance des fibres musculaires, par contre, en ouvrant le parapluie, celle-ci se rapproche.

Une telle sonde électro-biochimique peut être également associée à un dispositif de mesure de l'activité électroencéphalographique (EEG) et peut intégrer un dispositif de circulation d'un fluide biologique.

La sonde électro-biochimique de l'invention peut être également associée à un dispositif de mesure de l'activité électromyographique (EMG). Dans ce cas, le dispositif de l'invention permet d'enregistrer une activité électrique en surface grâce au dispositif de mesure de l'activité électromyographique (EMG), mais également de délivrer une drogue qui pénètre par voie transcutanée. Par ce dispositif, on peut confirmer qu'il existe une absorption hétérogène de la drogue lorsque le muscle présente des anomalies régionales ou que certains récepteurs font défaut sur certaines membranes cellulaires dans le muscle étudié. De plus, la partie de la sonde la plus proximale peut être maintenue en regard d'un tissu adipeux sous-cutané et l'on peut disposer d'informations concernant la distribution du médicament dans ledit tissu adipeux et dans le tissu musculaire proprement dit, en particulier, pour étudier la résorption transcutanée de molécules lipophiles.

Par ce dispositif, on peut également estimer très rapidement si la résorption intramusculaire est homogène ou hétérogène. Dans ce cas, la sonde électro-biochimique de l'invention comportera une chambre en contact avec le tissu adipeux sous-cutané et des compartiments ou chambres en contact avec le tissu musculaire.

Le dispositif de mesure de l'activité électromyographique (EMG) peut correspondre à celui décrit dans la demande de brevet WO2004/091389.

Les différents éléments de l'invention (sonde et dispositif incorporant cette sonde) peuvent trouver différentes applications dans le domaine de la recherche, mais également dans le domaine du traitement de différentes pathologies affectant l'homme ou l'animal. Par exemple, un patient humain développant une maladie des nerfs périphériques avec dégénérescence des axones moteurs (par exemple un syndrome de Guillain-Barré ou après une infection de poliomyélite) présente des signes de dénervation qui peuvent être détectés de manière non invasive, grâce à la sonde de l'invention.

En effet, la sonde électro-biochimique intramusculaire de la présente invention permet d'analyser les métabolites anormaux produits dans le muscle suite à une stimulation in loco des fibres musculaires.

De même, lors d'un accident de la route, un patient peut subir un écrasement d'une jambe. Si le membre est toujours mobilisable, il est possible d'infuser des liquides par voie intraveineuse, mais on ignore si cela ne va pas provoquer éventuellement un oedème des muscles lésés. La sonde électro-biochimique intramusculaire de l'invention permet de quantifier la production basale d'acide lactique et la réponse à une stimulation électrique in loco au niveau d'un muscle.

Le dispositif de l'invention permet d'analyser la réactivité électrique ou biochimique de fibres intramusculaires dans le territoire musculaire dépend du nerf sciatique et de comparer cette réactivité à celle des fibres intramusculaires dépendant d'un autre nerf (nerf crural) au niveau de la cuisse, par exemple. Pour étudier la réactivité, on va stimuler électriquement une ou plusieurs électrodes attachées à une ou plusieurs chambres de la sonde à des intensités croissantes. En cas de dénervation, on enregistre d'une part des fibrillations et d'autre part on observe un turn-over anormal d'acétylcholine. Lorsqu'un patient est opéré d'une « sciatique », le nerf sciatique peut être comprimé par une hernie. Malheureusement, la lésion du nerf sciatique est irréversible. Le patient développe une paralysie du pied, car les muscles releveurs du pied sont dénervés. Grâce à la sonde de l'invention, on peut suivre l'évolution de cette dénervation dans le temps. La sonde électro-biochimique intramusculaire va permettre d'analyser la « réactivité électrique et biochimique » des fibres intramusculaires dans le territoire musculaire dépendant du nerf sciatique et de comparer cette « réactivité » à celle des fibres intramusculaires dépendant d'un autre nerf (nerf crural au niveau de la cuisse, par exemple).

Si un accident de roulage survient, le conducteur peut être inconscient. On lui place un détecteur electro-encéphalographique (EEG) et un collier dynamique. Grâce au stimulateur intégré dans le détecteur (EEG), on peut tester la réactivité cérébrale. Pour cela, on couple le détecteur (EEG) avec quatre détecteurs electromyographique (EMG) (un par membre). On analyse par la sonde de l'invention aussi la production d'acide lactique de manière régionale dans les muscles des membres afin d'évaluer si le patient a développé un « syndrome des loges musculaires » régional, c'est-à-dire si le(s) muscle(s) qui a (ont) été traumatisé ont une production anormale de métabolites témoignant d'une compression des fibres musculaires dans la gaine (aponévrose) qui les entoure. Si tel est le cas, on enregistre la réactivité de plusieurs fibres à l'aide de la sonde électro-biochimique intramusculaire pour savoir si elles sont « sidérées », car il s'agit-là d'un index de mauvais pronostic.

On peut également disposer en continu des informations provenant d'une sonde électro-biochimique intramusculaire de l'invention sur l'état des fibres musculaires d'un patient hospitalisé. En effet, un traitement peut être infusé la nuit et on peut s'assurer que des potentiels électriques spontanés (fibrillations) n'apparaissent pas en cours de traitement chez le patient traité et surveillé.

Si un greffon musculaire est implanté sur un patient, la sonde de l'invention sur le patient permet de suivre le comportement électro-biochimique des fibres musculaires. Ces observations ouvrent de nouvelles perspectives en matière de compréhension de la pathogénie des maladies musculaires et de leur traitement.

Dans le cas où un patient présente des spasmes musculaires d'un hémicorps et une atrophie de l'hémicorps contra latéral suite à une maladie neurologique congénitale, on souhaite connaître les échanges métaboliques intramusculaires et les comparer pour chaque côté en vue d'administrer des traitements qui ne risquent pas d'aggraver ces spasmes. Grâce à deux sondes électro-biochimiques de l'invention (implantées respectivement à gauche et à droite, c'est-à-dire sur un membre gauche et un membre droit), une analyse pourra être réalisée au lit du malade ou même à la consultation de neurologie.

Pour un patient souffrant d'une maladie des motoneurones ou de la jonction neuromusculaire, il est utile d'obtenir une évaluation instantanée de la réponse des fibres musculaires à l'injection de plusieurs médicaments ou à des « cocktails de médicaments » (monothérapies versus polythérapies).

La sonde de l'invention trouve aussi des applications à l'étude in vivo de la vulnérabilité d'agents toxiques (comme l'éthanol) chez les patients suspectés de présenter des maladies enzymatiques et qui développent des symptômes inexpliqués suite (par exemple) à l'ingestion de petites quantités d'alcool, ou pour l'étude de la vulnérabilité à la cortisone dans la myopathie cortisonique ou la myopathie thyrotoxique.

En recherche clinique, il est important de pouvoir analyser par la sonde de l'invention, le comportement dans le temps des fibres musculaires dans une maladie évolutive comme la maladie de Werdnig-Hoffman ou la myasthénie gravis et la mise en évidence de nouveaux processus physiopathologiques non identifiés jusqu'à présent.

En outre, il est aussi possible de combiner les caractéristiques de la sonde et des dispositifs de l'invention avec d'autres moyens permettant une exploration de la fonction électro-biochimique des fibres musculaires soumises à des vibrations de très haute fréquence. Ces moyens peuvent, par exemple, constituer en un dispositif d'induction volontaire de mouvements selon la technique haptique, telle que décrite dans la demande de brevet PCT/BE2005/000090. En effet, ces vibrations induisent des contractions réflexes des fibres musculaires.

Il est aussi possible d'étudier la « barrière musculo-sanguine » d'un animal ou d'un patient par la sonde de l'invention, suite à une infusion intramusculaire (dans un muscle soumis à une stimulation électrique répétée) d'un médicament A et en l'étude de la cinétique du médicament A ou de ses métabolites dans un autre muscle qui lui est au repos et d'identifier ainsi des malades suspects de présenter une fragilité des capillaires intramusculaires.

De plus, une ou plusieurs fibres optiques peuvent être insérées dans chacun des compartiments de la sonde électro-biochimique de l'invention afin d'obtenir une « visualisation intramusculaire in vivo » (VIMIV) des phénomènes de contraction musculaire. Il s'agit d'une technique permettant de visualiser les fibres musculaires à chacun des étages de la sonde électro-biochimique.

## Revendications

1. Une sonde de microdialyse intramusculaire ou intracérébrale avec un guide (1), comprenant :
- au moins une chambre (2) avec des pores (5), ladite chambre (2) étant connectée à un élément d'infusion (3) d'un liquide vers ladite chambre (2) et un élément d'aspiration (4) dudit liquide de ladite chambre (2) et,
- des électrodes d'enregistrement (7,8) et de stimulation (9) capables de délivrer des différences de potentiel continu.

2. La sonde selon la revendication 1, comprenant également des stimulateurs électriques, des préamplificateurs et des filtres.

3. La sonde selon la revendication 1 ou 2, dans laquelle le guide (1) comprend plusieurs chambres (2, 2', 2"), chacune étant connectée à des éléments d'infusion (3) et d'aspiration (4) différents.

4. La sonde selon la revendication 3, dans laquelle les chambres (2,2',2") sont formées par des prismes ou cylindres présentant une section d'un diamètre décroissant et insérés les uns dans les autres.

5. La sonde selon la revendication 4, dans laquelle les prismes ou cylindres insérés les uns dans les autres sont aptes à coulisser les uns par rapport aux autres.

6. La sonde selon la revendication 3 à 5, dans laquelle chaque chambre (2, 2', 2") comporte des pores (5) de taille différente d'une chambre à l'autre.

7. La sonde selon les revendications 3 à 6 dans laquelle la chambre (2,2', 2") comporte différents pores de taille différente.

8. La sonde selon l'une quelconque des revendications précédentes, comportant également une ou plusieurs fibres optiques présentes dans une ou plusieurs chambre(s) et/ou un senseur de variation de la température.

9. Un dispositif comprenant plusieurs sondes selon l'une quelconques des revendications précédentes, connectées entre elles et comportant un élément créateur d'un champ magnétique formé à côté d'au moins une des chambres (2) d'une des sondes.

10. Le dispositif selon la revendication 9, dans lequel l'élément créateur du champ magnétique consiste en un courant électrique induit dans des spires (14) présentes dans la chambre.

11. Un dispositif comprenant une ou plusieurs sondes, selon l'une quelconque des revendications précédentes 1 à 8, un élément (10) d'amplification et de filtrage d'un signal électrique provenant des électrodes d'enregistrement (7,8), un élément (11) d'analyse de données biochimiques spécifiques obtenues sur le liquide d'aspiration de ladite chambre (2) de la sonde et un écran permettant un affichage simultané du signal électrique (A) et des données biochimiques (B) obtenues de la sonde.

12. Le dispositif selon la revendication 11, combiné à un dispositif d'analyse d'un électroencéphalogramme (EEG).

13. Le dispositif selon les revendications 11 ou 12 combiné à un dispositif d'analyse d'un électromyogramme (EMG).
